# NEW EUROPEAN PATENT SPECIFICATION

(11) **EP 0 399 564 B2**
(45) Date of publication and mention of the opposition decision: **24.04.2002**
(45) Mention of the grant of the patent: 27.07.1994
(21) Application number: 90110090.9
(22) Date of filing: 28.05.1990
(51) Int. Cl.: A61L 15/00

(54) **Absorbent web and applications of same**
Absorbierende Schicht und deren Verwendung
Couche absorbante et utilisation de celle-ci

(30) Priority: 26.05.1989 US 358919
(43) Date of publication of application: 28.11.1990
(73) Proprietor: KIMBERLY-CLARK CORPORATION, Neenah, Wisconsin 54956-0349 (US)
(72) Inventor: Bernardin, Leo J., Appleton, WI 54911 (US); Heimbach, Catherine J., Stockbridge, WI 53088 (US)
(74) Representative: Diehl, Hermann O. Th., Dr.

(56) References cited:
- EP-A- 0 209 884
- WO-A-88/04704
- GB-A- 2 208 477
- US-A- 3 241 553
- US-A- 3 658 613
- US-A- 3 971 379
- US-A- 4 252 761
- US-A- 4 256 111
- US-A- 4 767 848
- Proceedings, Tappi, Annual Meeting (Chicago), 133-140 (March 2-5, 1981), Martinis et al

## Description

The present invention relates to absorbent structures formed from cellulose fibers.

In absorbent products, such as diapers, incontinence garments and the like, a body liquid, such as urine, is generally locally applied to an absorbent pad present in the absorbent product. The capacity to absorb and store the liquid in the absorbent product depends, in large part, upon the physical properties of the material from which the absorbent pad is formed. Accordingly, much effort has been devoted to improving the fluid absorbing properties of the materials from which the absorbent pads are fabricated. The materials from which the absorbent pads are fabricated include, wood pulp fluff, cotton, cotton linters and other cellulosic fibers.

Wet crosslinking of cellulose material is discussed in U.S. Patent No. 3,320,956 issued May 23, 1967 to Steiger. Steiger describes wet crosslinking of cellulose fibers to increase the wet resiliency of the fibers and the fluid retention of webs formed from the fibers. Wet crosslinking of cellulose fibers is described as producing an improvement in the fluid absorbency, fluid retention and wet and dry resilience properties of an absorbent material that is used in tampons.

International Patent Cooperation Treaty ("PCT") application WO88/04804 published June 30, 1988 is directed to a method of making a hydrophilic cellulosic pulp. The method involves treating a wet-laid fibrous web with an aqueous solution of glycol and dialdehyde followed by drying. The treated webs are described as having an increased absorbency rate and an improved water-holding capacity.

International PCT application WO85/3509 published August 15, 1985 describes modified polysaccharide materials. Polysaccharide material, such as wood pulp, is treated with N, N'-methylenebisacrylamide. The modified polysaccharide is described as possessing increased bulk and absorbency.

Wet crosslinking of cellulose pulp is known for use in the manufacture of paper to produce a paper having increased porosity and absorbency. This is disclosed in U.S. Patent No. 3,069,311 issued December 18, 1962 to Harpham et al.

U.S. Patent 3,658,613 issued April 25, 1972 to Steiger is directed to a method for wet crosslinking pulp board. The wet crosslinked pulp board can be formed into a pulp fluff which possesses a reduced knot content, improved wet resiliency, and increased fluid absorption and retention capacity.

WO 88/04704 discloses an absorbent web made by a process similar to a dry crosslinking process. However, the final cuting process of the typical dry crosslinking process is not performed in the process disclosed in WO 88/04704.

Unfortunately, none of the references discussed above describe or suggest the critical physical characteristics necessary to produce an absorbent pad generally capable of distributing a liquid applied locally thereto throughout a majority of its structure. Specifically, none of the references describe or suggest the critical nature of selecting the density of an absorbent web and the manner of crosslinking the fibers of such a web as they relate to the vertical wicking properties of the web.

The invention provides a web according to independent claims 1 and 5. Further advantageous features of the web are evident from dependent claims 2 to 4 and 6 to 8 and the following description and drawings. The invention also teaches use of the web as an absorbent product, especially as a diaper (according to claim 9). The invention also provides an absorbent product according to independent claim 10. Further advantageous features of this product are evident from dependent claims 11 and 12 and the following description and drawings. The invention further provides a diaper according to independent claim 13. Further advantageous features of the diaper are evident from dependent claims 14 to 17 and the following description and drawings. The claims are to be understood as a first non-limiting attempt to define the invention in general terms.

The invention provides vertical wicking structures from wet crosslinked cellulose fiber structures. Specifically, the present invention relates to absorbent structures formed from wet crosslinked cellulose fibers which absorbent structures possess improved vertical wicking properties.

In a first aspect, it is desirable to produce an absorbent web formed from cellulose fibers which absorbent web possesses improved vertical wicking properties such that the absorbent web is capable of rapidly distributing a liquid applied locally thereto throughout its structure. Additionally, it is desirable to produce an absorbent web having improved wet resiliency.

In a second aspect, it is desirable to provide an absorbent web for use as a component in an absorbent product such as an incontinence product, feminine napkin, absorbent dressing, or the like which possesses enhanced fluid distribution properties and thereby improves performance of the absorbent product.

These and other related goals are achieved in an absorbent web formed from wet crosslinked cellulose fibers which absorbent web has a density within the range of from about 0.08 grams per cubic centimeter to about 0.35 grams per cubic centimeter. Such an absorbent web has been found to possess improved vertical wicking properties and enhanced wet resiliency. In a first embodiment, the absorbent web according to the present invention is produced by airlaying mats of wet crosslinked cellulose fibers which air-laid mats are then compressed to form absorbent webs having a density within the range of from 0.10 to 0.35 grams per cubic centimeter. In a second embodiment, the absorbent webs according to the present invention are produced by water laying wet crosslinked cellulose fibers into an absorbent web having a dry density within the range from 0.08 grams per cubic centimeter to 0.35 grams per cubic centimeter.

Figure 1 is a graphical representation of the vertical wicking rate data set forth in Table 1.

Figure 2 is a bar graph of the fluid distribution data set forth in Table 1.

Figure 3 is a graphical representation of the vertical wicking rate data set forth in Table 3.

Figure 4 is a graphical representation of the vertical wicking rate data set forth in Table 4.

Figure 5 is a bar graph of the fluid distribution data set forth in Table 3.

Figure 6 is a bar graph of the fluid distribution data set forth in Table 4.

Figure 7 is a graphical representation of the vertical wicking rate data set forth in Table 5.

Figure 8 is a bar graph of the fluid distribution data set forth in Table 5.

Figure 9 is a graphical representation of the vertical wicking rate data set forth in Table 6.

Figure 10 is a bar graph of the fluid distribution data set forth in Table 6.

Figure 11 is a graphical representation of the vertical wicking data of Example 5.

Figure 12 is a bar graph of the fluid distribution data set forth in Table 7.

### DETAILED DESCRIPTION OF THE PREFERRED EMBODIMENTS

Present invention relates to an absorbent web of wet crosslinked cellulose fibers which web has a density, under a load of 0.0138 bar (0.2 pounds per square inch), within the range of from 0.08 to 0.35 grams per cubic centimeter which web displays improved vertical wicking properties compared to similar webs that are not wet crosslinked.

Reference to a "similar web" which is non wet crosslinked is intended to refer to a web having the same physical characteristics, (e.g. density, fiber length, denier, basis weight) and which is formed in the same manner (air-laying, water-laying, carding, etc.). In essense, the only distinction between the webs is that one is wet crosslinked and the other is not.

As used herein the term "vertical wicking properties" is intended to include, without limitation, vertical wicking rate, vertical wicking capacity, and vertical fluid distribution. Vertical wicking rate refers to the rate at which fluid is being vertically wicked over a given time period. Vertical wicking capacity refers to the total amount of fluid absorbed in a given time period. Vertical fluid distribution refers to the amount of liquid vertically wicked to a given height in a given time period. These properties will be discussed in greater detail below in connection with the examples.

A variety of cellulosic fibers are known to those skilled in the art. It is believed that any natural cellulosic fiber material capable of forming fibrous webs is suitable for use in the present invention. Exemplary of cellulosic fibers suitable for use in the present invention are natural cellulosic fibers such as cotton; wood pulp; bast fibers such as flax and jute; stem or leaf fibers such as abaca, sissal, bagasse; and the like. Methods of pre-paring the cellulosic fibers suitable for use in the present invention are known to those skilled in the art. For example, wood pulp is generally prepared by fiberizing sheets or strips of wood pulp board in hammermills or other commercial shredding devices into fluff which can then be formed into webs.

According to the present invention, the cellulosic fibers are wet crosslinked. As used herein, the term "crosslinked" is intended to refer to the situation in which two chains of cellulose molecules are joined together by chemical bridges.

Methods of crosslinking cellulose are known to those skilled in the art. As a general rule, such methods involve contacting the cellulose fibers with a crosslinking agent. A crosslinking agent is an agent capable of combining with at least two hydroxyl groups in the cellulose molecule or in adjacent cellulose molecules. In order to crosslink cellulose, the crosslinking agent is preferably at least bifunctional with respect to cellulose; e.g., it must react with at least two hydroxyl groups. Any crosslinking agent capable of performing in the described manner is suitable for use in the present invention. Exemplary of crosslinking agents known for use with cellulosic fibers are formaldehyde; 1,3,dichloro-2-propanol, methylenebisacrylamide; condensation products of formaldehyde with organic compounds such as urea, or the like; dialdehydes; diepoxides; diisocyanates; divinyl compounds; other, di-halogen containing compounds such as dichloroacetone; halohydrins such as epichlorohydrin; and the like. It is to be understood that some of these agents may form polymeric crosslinks. The preferred crosslinking agent is selected from the group consisting of formaldehyde, 1,3-dichloro-2-propanol,and methylenebisacrylamide.

According to the present invention, the cellulose fibers are crosslinked while in a swollen state (wet crosslinking). As a general rule, the cellulosic fibers will be swollen with water; however, it is to be understood that it is also possible to swell the cellulosic fibers with solvents other than water. It is generally desirable to use water for economic and safety reasons.

When water is used to swell the cellulosic fibers, it is generally desirable that the cellulosic fibers contain at least 30 percent by weight of water. It is generally preferred that the cellulose fibers be completely water-swollen; i.e., that the cellulose fibers be swollen to the greatest extent possible by placing the fibers in an excess of an aqueous solution until the fibers are saturated, or in an aqueous alkali solution for greater swelling, where suitable for the reaction.

The crosslinking occurs when a crosslinking agent is brought into contact with the swollen cellulosic fibers. As a general rule, the crosslinking reaction occurs by placing the cellulosic fibers into a solution containing the crosslinking agent. The cellulosic fibers are allowed to remain in the solution until the desired degree of crosslinking is obtained.

After achieving the desired degree of crosslinking, the cellulose fibers are removed from the crosslinking solution and washed several times to remove all of the crosslinking solution from the cellulosic fibers. The crosslinked cellulosic fiber is then formed into fibrous webs.

Fibrous webs according to the present invention can be formed, for example, by air-laying or wet-laying the wet crosslinked cellulosic fibers. Specifically, the wet crosslinked cellulosic fibers can be dried, fiberized, air-laid and compressed to the desired density. Alternatively, the wet crosslinked cellulosic fiber can be suspended in an aqueous solution and wet-laid.

Applicants have discovered that in order to obtain the desired vertical wicking properties, it is necessary for the wet crosslinked cellulosic fibers to be formed into webs having a density of from 0.10 to 0.35 grams per cubic centimeter preferably, from 0.15 to 0.3 grams per cubic centimeter for air-laid webs and from 0.08 to 0.35 grams per cubic centimeter, preferably, from 0.08 to 0.30 grams per cubic centimeter for water-laid webs.

When the fibrous webs are air-laid, it is generally necessary to compress the fibers to achieve the desired density (fine pore structure). However, when the fibrous webs are formed through a wet-laying process, it is generally not necessary to compress the webs to achieve the desired density. The airlaid webs can be compressed, for example, in a press such as can be obtained from Dake, Grand Haven, Michigan as a "Dake Laboratory Press", Model No. 44-148. Alternatively, a heated calendar nip is suitable for use to compress the fibers to the desired density. The webs can be compressed with or without the application of heat.

Applicants have discovered that webs according to the present invention possess improved vertical wicking properties. These improved properties are desirable when the webs are intended for use as an absorbent material in an absorbent product such as a diaper, a feminine napkin, a surgical dressing, and the like.

As a general rule, the vertical wicking properties of a fibrous web according to the present invention will be considered to be improved when the web exhibits at least a 20 percent increase in initial vertical wicking rate (average rate, 3-21 seconds as determined from the slope of the vertical wicking curve as discussed in more detail below), vertical- wicking capacity (at 15 or 30 minutes) or vertical fluid distribution (at a distance of eighteen (18) centimeters) compared to a similar web of non wet crosslinked fibers. Preferably, the webs according to the present invention exhibit one or more vertical wicking property(ies) which is 40 percent greater than that property or properties exhibited by the similar, non wet crosslinked web to which it is compared. Additionally, it is most preferred that the webs according to the present invention demonstrate at least a 20 percent and preferably a 40 percent increase in at least two vertical wicking properties when compared to the similar web of non wet crosslinked fibers.

When the fibrous webs according to the present invention form the absorbent material in a diaper, the liquid which is intended for absorption into the diaper is generally applied to the diaper in a fairly localized region. Nonetheless, the absorbent material is generally located throughout the body of the diaper. It is, therefore, important that the absorbent material be capable of wicking the liquid applied to the diaper away from the position at which it is applied into absorbent material located remote from the application location. In this manner, a greater extent of the absorbent material present in the diaper is utilized. By the very nature of the use of diapers, it is generally necessary that the diaper be capable of wicking liquid not only in a horizontal direction, but, more importantly, also in a vertical direction in order to utilize the greatest amount of the absorbent material present in the diaper.

In those instances in which the absorbent webs of the present invention are used in a diaper, the diaper will generally comprise an outer, water-impervious layer having adjacent to one surface thereof the web of the instant invention which in turn has adjacent to its surface a water-pervious body liner adapted to contact the skin of a wearer. Such a diaper is described in greater detail in U.S. Patent Nos. 4,710,187 issued December 1, 1987 to Boland et.at.; 4,762,521 issued August 9, 1988 to Roessler et al; 4,770,656 issued September 13, 1988 to Proxmire et al; and 4,798,603 issued January 17, 1989 to Meyer et al., which references are incorporated herein by reference.

In some instances it may be desired to incorporate into the webs of the present invention, or in flow communication therewith, an amount of a water-swellable polymer material. The water-swellable polymeric material is considered to be in flow communication with the web when liquid present in the web can flow into contact with the polymeric material. For example, the water-swellable polymer may be located directly in the web or may be in a seperate pad or layer adjacent the web and adapted to allow fluid to move from the web into the pad or layer. Water swellable polymeric materials are known to those skilled in the art. Exemplary of water-swellable polymeric materials suitable for use in the present invention are polyacrylamides, polyvinyl alcohol, ethylene maleic anhydride copolymers, polyvinyl ethers, hydroxypropyl cellulose, carboxymethyl cellulose, polyvinylmopholinone, polymers and copolymers of vinyl sulfonic acid, salts of polyacrylic acid, which have been insolubilized by crosslinking or pseudo-crosslinking as in chain entanglement, starch grafted polyacrylates, polyvinyl pyridine, and the like.

The use of water-swellable polymeric materials in diapers is known to increase the absorptive capacity of the diaper. Unfortunately, when the water-swellable polymeric material is present in areas of the diaper remote from the location at which the liquid to be absorbed is applied to the diaper, the water-swellable polymer does no good if the liquid does not reach it.

As discussed above, the webs of the present invention possess improved vertical wicking properties and are, therefore, better able to distribute a liquid throughout their structure. Thus, when water-swellable polymers are incorporated into, or in capillary contact with, the webs of the present invention, the improved vertical wicking abilities of the webs allows them to more readily transport the liquid to the water-swellable polymer located remote from the area of application. An improved diaper is thus provided.

The present invention can best be understood by reference to the following examples (including comparative examples).

### EXAMPLES

In all of the following examples, including comparative examples, the vertical wicking properties are determined in the following manner.

A piece of the absorbent web is cut into a 7.62 cm (3 inch) by 38.1 cm (15 inch) test sample. The test sample is mounted on a Lucite² plate which is 0.95 cm (3/8 inch) thick, 33.02 cm (13 inches) wide and 35.56 cm (14 inches) long. The sample to be tested is symmetrically wrapped around the edge of the plate with the longitudinal dimension of the test sample parallel to the longitudinal dimension of the plate. The opposite longitudinal ends of the sample are fastened to the plate by a spring-loaded clip or clamp. The sample is supported on the outside of the plate by a ten-mesh nylon net.

The plate is suspended vertically over a fluid bath with the longitudinal dimension of the sample perpendicular to the fluid's surface. The fluid is brought into contact with the sample by immersing the lower edge of the plate into the fluid such that the portion of the sample bending around the edge of the plate is slightly immersed. The amount of fluid absorbed as a function of time is measured at serveral time intervals for the 30-minute duration of the test. The sample is then removed from fluid contact. The sample is removed from the plate and weighed, and the fluid pick-up noted. After weighing, the sample is laid horizontally on a 7.62 cm (3 inch) by 38.1 cm (15 inch) cutting die which is segmented into nine zones of about 4.32 cm (1.7 inches) each. The cutting die includes cutting edges across the die width and around the outer perimeter. Pressure applied to the die divides the sample along its length into nine almost identically sized segments. Each segment is weighed, oven-dried and reweighed, and the fluid pickup determined on a grams of fluid per gram of fiber basis (corrected for deposited solids from the fluids).

The amount of fluid absorbed is calculated as the milliliters of fluid-per-unit basis weight (gram per square centimeter) per unit width (2.54 cm (1 inch)). The vertical wicking capacity is defined as the amount of fluid absorbed in the defined units at the end of 15 and 30 minutes. The vertical wicking rate is measured as the slope of the plot of the amount absorbed/unit basis weight/unit width versus the square root of the time (seconds) over the time interval from 1 to 30 seconds. The initial vertical wicking rate is the average rate from 3-21 seconds in the linear portion of the curve. The fluid distribution is determined from the measured fluid pickup of the 7.62 cm (3 inch) by 4.2 cm (1.7 inch) segments prepared as described above, and is reported as a function of distance from the point of contact with the liquid.

The fluid employed is a synthetic urine comprising 0.31 grams monobasic calcium phosphate monohydrate (CaH₄(PO₄)₂ H₂O), 0.68 grams monobasic potassium phosphate (KH₂PO₄), 0.48 grams magnesium sulfate heptahydrate (MgSO₄ x 7H₂O), 1.33 grams potassium sulfate, (K₂SO₄) 1.24 grams tribasic sodium phosphate dodecahydrate (Na₃PO₄ x 12H₂O), 4.4 grams sodium chloride (NaCl), 3.16 grams potassium chloride (KCI), 0.4 grams sodium azide (NaN₃), 8.56 grams of urea (CO(NH₂)₂) and 0.1 gram Pluronic 10R8 surfactant (a nonionic surfactant commercially available from BASF-Wyandotte Corporation) per liter using distilled water as the solvent. The components are added to 900 milliliters of distilled water in the order given and each dissolved before the next component is added, the solution is finally diluted to one liter.

### Example 1

### Sample 1

Bleached southern pine kraft pulp (75 percent pine softwood and 25 percent hardwood) is provided. The southern pine kraft pulp is then wet crosslinked using formaldehyde as the crosslinking agent in the following manner. One hundred grams (100g) of the fiberized pulp is added to 500 milliliters of a solution containing, by volume, 20-percent formalin, 50-percent hydrochloric acid (37%) and 30-percent distilled water. After mixing, the pulp-containing solution is allowed to stand at room temperature for 30 minutes. The pulp is then removed by filtration, washed twice with an excess of distilled water, neutralized with an aqueous sodium carbonate solution (5%) and washed three more times with an excess of distilled water. The crosslinked pulp is then air-dried.

### Sample 2

A second sample of the southern pine kraft pulp is wet crosslinked using 1,3,dichloro-2-propanol (DCP) as the crosslinking agent in the following manner. One hundred grams of pulp is placed in a plastic bag. To the bag is added 200 milliliters of distilled water in which 20 grams of 1, 3-dichloro-2-propanol has been dissolved. The contents of the bag are massaged to evenly distribute the liquid. To the bag is then added 200 milliliters of distilled water in which 20 grams of sodium hydroxide has been dissolved. The contents of the bag are again thoroughly mixed. The bag is sealed and allowed to stand at room temperature for 24 hours. The pulp is recovered by filtration and rinsed with distilled water until the filtrate tests neutral. The pulp is then oven dried at 60 °C overnight.

### Sample 3

A third sample of the southern pine kraft pulp is dry crosslinked using a low-formaldehyde modified glyoxal based resin commercially available from American Hoechst under the trade designation Cassurit NDS with Cassurit AM catalyst according to the following procedure. To 232 grams of distilled water is added, with stirring, 13.3 grams of Cassurit NDS and 4.6 grams Cassurit catalyst AM. To the solution is then added 100 grams of pulp. The resulting slurry is then oven dried at 75°C. After drying, the pulp is fiberized and cured at 150°C for 15 minutes. The pulp is then washed thoroughly with distilled water to remove any unreacted resin or catalyst and is then oven dried at 60 ° C.

After completion of the above-described crosslinking procedures, all of the pulps are air-dried, fluffed, and air-laid on a hand-sheet former to form fibrous webs. Additionally, a control sample of non-crosslinked southern kraft pulp is air-laid on a handsheet former to form a fibrous web. The air-laid webs are then densified to a density of 0.15 grams per cubic centimeter in a heated press such as can be obtained from Dake, Grand Haven, Michigan under the trade designation "Dake Laboratory Press" Model No 44-148.

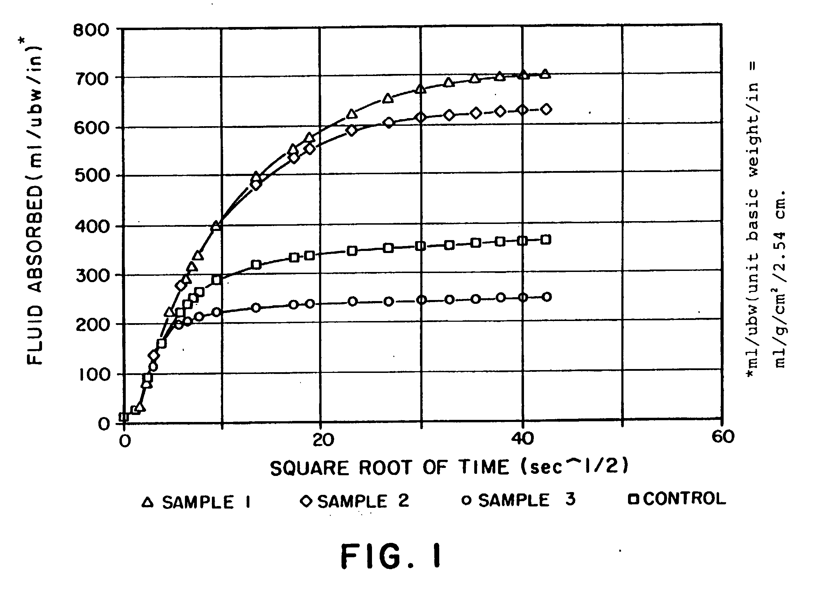

The four test samples described above are then subjected to the vertical wicking tests described above. The results of the vertical wicking tests are set forth in Table 1. Figure 1 graphically illustrates the results of the vertical wicking rate determination.

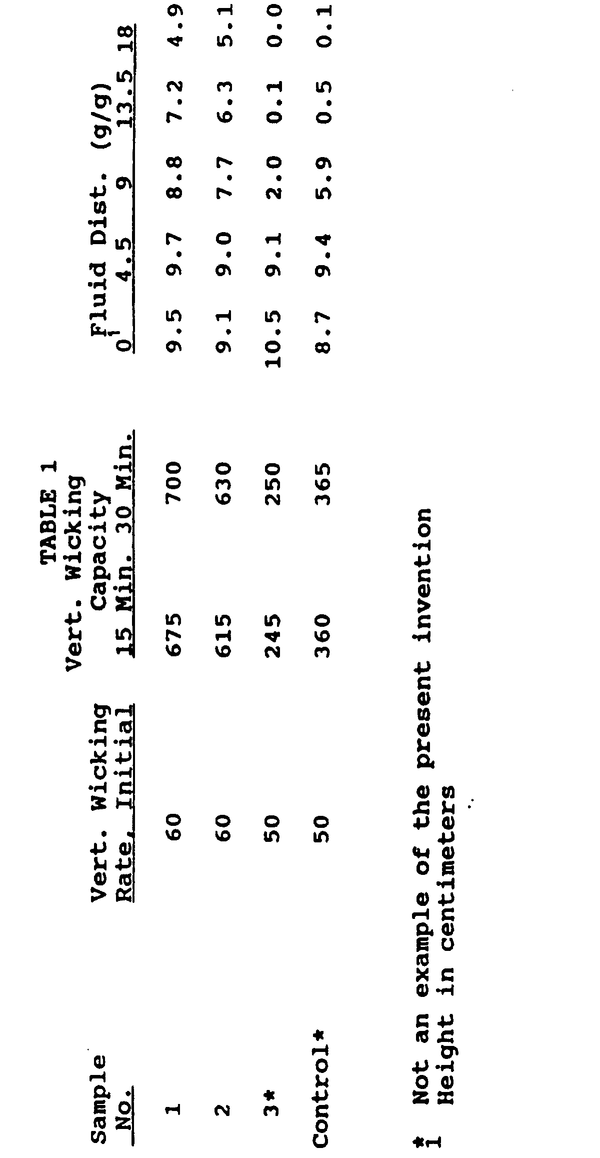

As can be seen from reference to Figure 1 and Table 1 with respect to vertical wicking properties, the wet crosslinking of the southern kraft pulp with either formaldehyde or 1,3,dichloro-2-propanol produces significantly improved performance compared to a non-crosslinked southern kraft pulp or a dry crosslinked southern kraft pulp. In fact, it is seen that dry crosslinking actually impedes the vertical wicking rate of the southern kraft pulp.

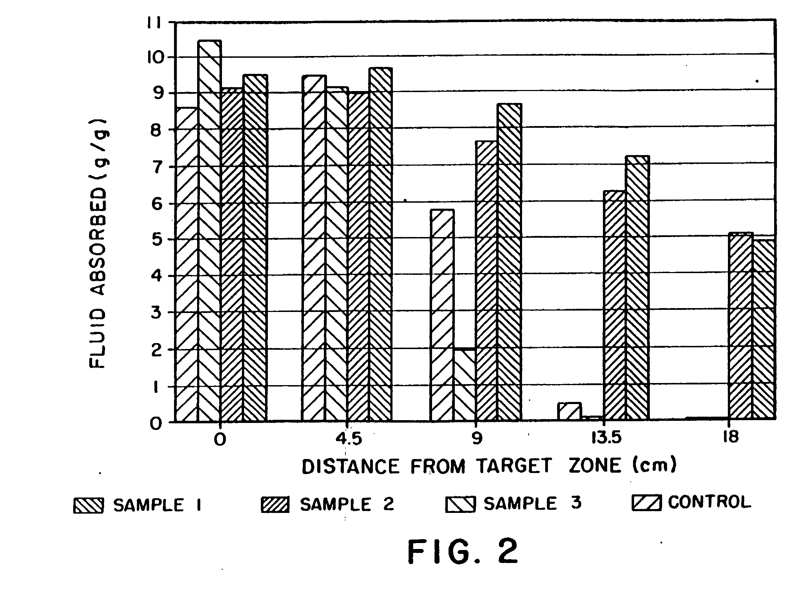

Figure 2 is a bar graph illustrating the fluid distribution data presented in Table 1. As can easily be seen from reference to Figure 2, all of the test samples are generally equivalent at low vertical wicking distances. However, at distances between about 9 and 18 centimeters the wet crosslinked samples significantly outperform either the dry crosslinked or the non crosslinked samples.

The dry and wet specific volumes of the four test samples described above are then determined. For measuring the dry and wet specific volumes of the sample pulps, an Ames thickness gauge (No.3222) capable of measuring thicknesses to 0.025 mm (0.001 inch) is employed. The thickness gauge is mounted above a demand absorbency tester as described in U.S. Patent 3,952,548 issued April 27, 1976 to Lichstein.

Test pieces, having basis weight between 200-300 grams per square meter, of the webs of Samples 1-3 and the control sample are prepared by cutting a 7.62 cm (3 inch) diameter disc from the densified webs with a 7.62 cm (3 inch) diameter die. The demand absorbency test apparatus is adjusted to a zero hydrostatic head using a saturated filter paper above the admittance port. The thickness of the dry sample is measured with the thickness tester, which sample is insulated from the fluid by a plastic disc, at a foot pressure of 0.0138 bar (0.2 pounds per square inch).

The plastic disc is removed and the test sample located on the filter paper (after first re-zeroing the thickness gauge) with a 0.0035 bar (0.05 pounds per square inch) compressive load. The sample is then saturated with synthetic urine at zero hydrostatic head until no further dimensional change occurs.

The foot pressure is then increased to 0.0138 bar (0.2 pounds per square inch) and the thickness measured at equilibrium. From the basis weight and the measured dry and wet thicknesses at 0.0138 bar (0.2 pounds per square inch), the respective specific volumes can be calculated.

The results of this test are set forth in Table 2.

**TABLE 2**

| SAMPLE NO. | SPECIFIC VOLUME¹ | | PERCENT CHANGE |
|---|---|---|---|
| | DRY | WET | |
| 1 | 6.7 | 9.9 | 47 |
| 2 | 6.5 | 9.7 | 49 |
| 3 | 6.8 | 9.3 | 36 |
| CONTROL* | 6.3 | 8.3 | 31 |

| | | | |
|---|---|---|---|
| * Not an example of the present invention. | | | |
| 1) Specific volume in cubic centimeters per gram under a 0.0138 bar (0.2 pound-per-square-inch) load. | | | |

The specific volume is an indicator of the wet resiliency of a given test sample. That is, a comparison between the specific volume of a given sample when dry and the same sample when wet directly corresponds to the wet resiliency of the sample. As can be seen from reference to Table 2, the percent change between the dry specific volume and the wet specific volume for the wet crosslinked samples is significantly greater than for either the dry crosslinked sample or the noncrosslinked control sample. Thus, the wet crosslinked samples are possessed of a greater wet-resiliency than the dry crosslinked sample or the non-crosslinked control sample.

### EXAMPLE 2

Bleached northern spruce kraft pulp is subjected to a wet crosslinking process using 1,3,dichloro-2-propanoi as the crosslinking agent. The wet crosslinking process is as follows:
To 900 milliliters of distilled water is added 50 grams of sodium chloride, 50 grams of sodium hydroxide, and 100 grams of 1,3,dichloro-2-propanol. The resulting solution is then diluted with distilled water to make one liter of solution. To the resulting solution is added 100 grams of bleached northern spruce kraft pulp (fiberized) to form a slurry. The slurry is stirred and allowed to stand at room temperature for 15 hours. The pulp is recovered by filtration and washed six times with an excess of distilled water, until the filtrate is free of alkali and 1,3,dichloro-2-propanol. The wet crosslinked pulp is then carefully air-dried at room temperature. The air-dried pulp is then fiberized in a pulverizer, commercially available from Pallman under the trade designation Pallman pulverizer, air-laid in an airlaying handsheet former, and densified to various densities within the range of from 0.05 to 0.3 grams per cubic centimeter in 0.05 grams-per-cubic-centimeter intervals. Six samples so prepared are then subjected to vertical wicking property determinations as described above. The vertical wicking properties of the samples are set forth in Table 3.

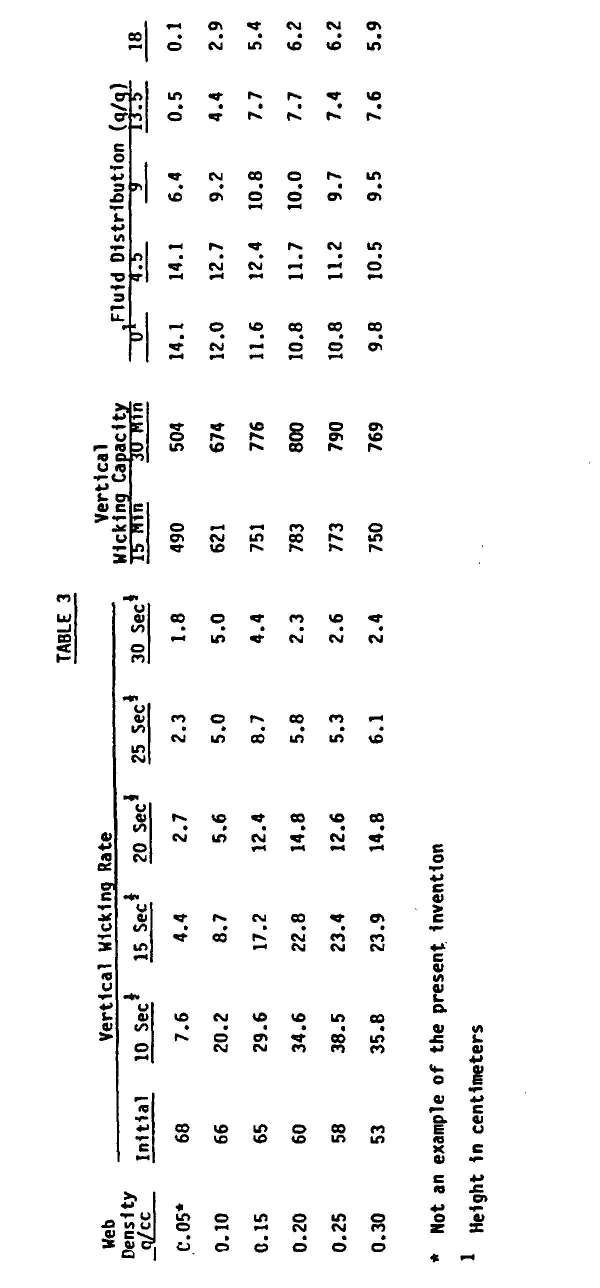

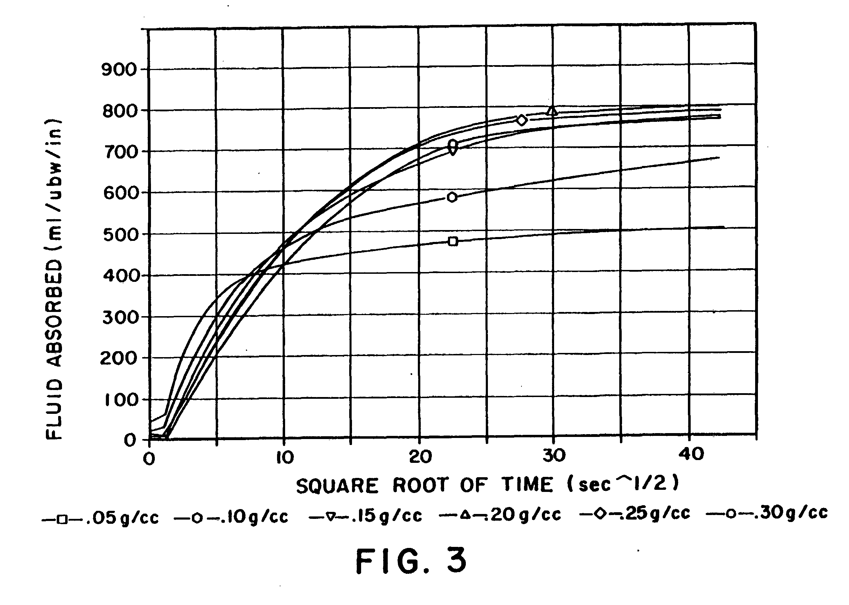

The vertical wicking rates set forth in Table 3 are graphically illustrated in Figure 3.

A control sample of non-crosslinked northern spruce kraft is similarly air-laid, ensified to a variety of densities, and subjected to vertical wicking property testing. The results of these tests are set forth in Table 4.

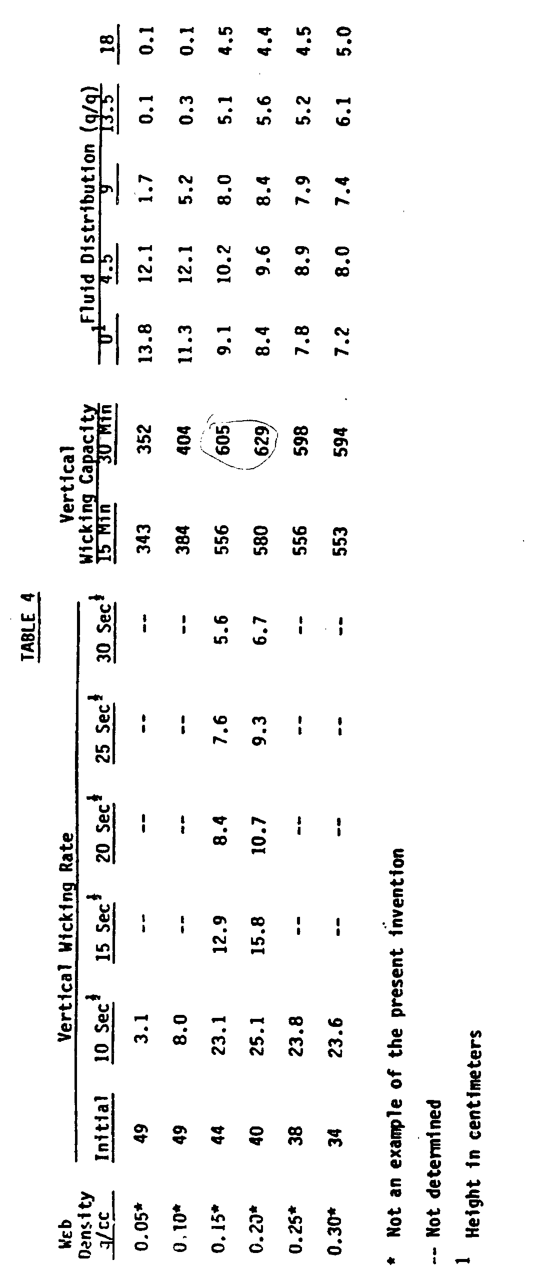

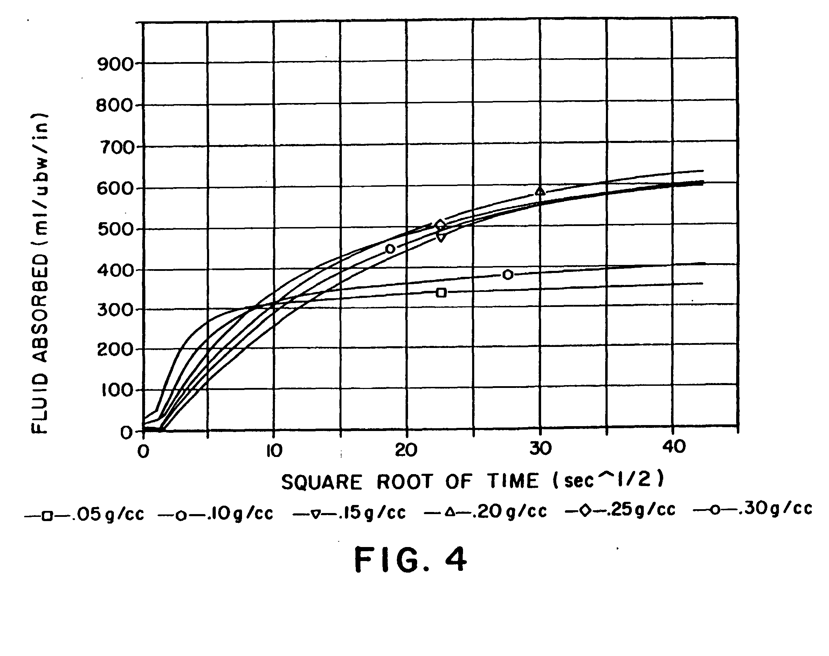

The vertical wicking rate data set forth in Table 4 are graphically illustrated in Figure 4.

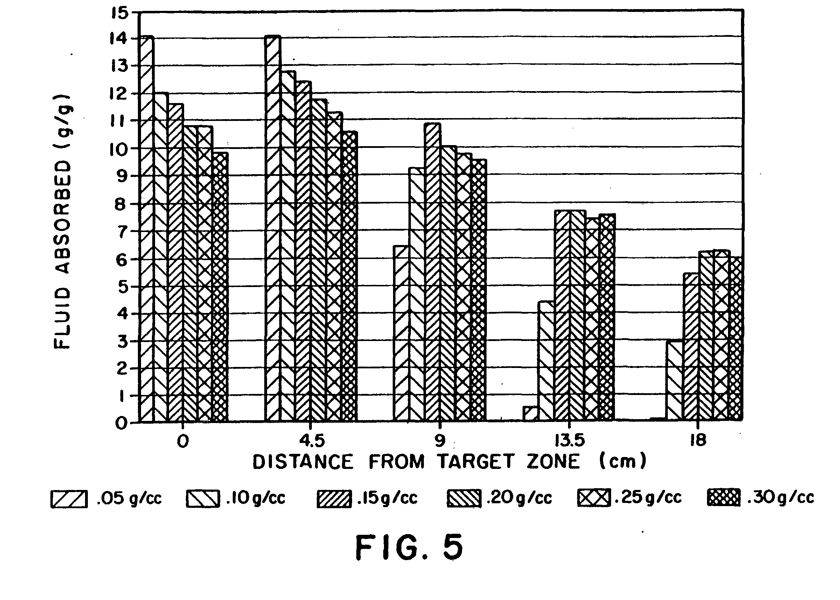

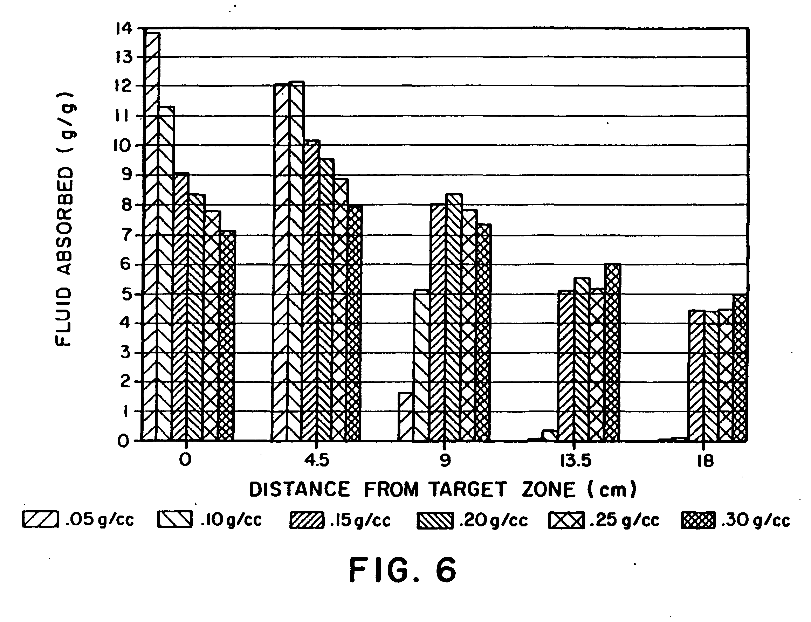

Figure 5 is a bar graph of the fluid distribution data set forth in Table 3. Similarly, Figure 6 is a bar graph of the fluid distribution data for the control sample set forth in Table 4.

As can be seen from reference to Table 3 and Figure 3, the vertical wicking capacity increases as density increases to obtain a maximum at a density of 0.20 grams per cubic centimeter and begins to fall off as the density increases beyond the 0.20 grams-per-cubic-centimeter level. Additionally, the vertical wicking rates are, initially, lower at the higher densities. However, it is seen that the vertical wicking rates for the higher density mats cross over those of the lower density webs, resulting in higher subsequent vertical wicking rates for the higher density webs.

Reference to Figure 5 reveals that the lower density webs have superior fluid distribution properties at 0 and 4.5 centimeter distances. However, for the distance between 9 and 18 centimeters, it is seen that densities above 0.15 grams per cubic centimeter are generally superior.

With respect to the non-crosslinked control sample, Figure 4 illustrates the same general pattern as discussed in connection with Figure 3 except at lower overall vertical wicking rates. Additionally, Figure 6 illustrates that higher densities are again preferred to obtain better fluid distribution at distances between 9 and 18 centimeters again, at lower overall values.

### Example 3

Northern spruce kraft pulp is wet crosslinked with 1,3,dichoro-2-propanol in the following manner. One hundred grams of northern spruce kraft pulpboard is dispersed in distilled water and filtered. The amount of water retained in the pulp is determined by weighing. A crosslinking agent solution is made by dissolving, in 700 milliliters of distilled water, 50 grams of sodium hydroxide, 50 grams of sodium chloride, and 100 grams of 1,3,dichloro-2-propanol. The wet pulp is then added to the crosslinking agent solution which is then diluted to one liter, including the amount of water retained by the pulp, with distilled water.

The crosslinking agent solution and wet pulp are placed in a plastic bag and thoroughly kneaded together. The slurry is allowed to stand at room temperature for 18 hours. The pulp is then recovered by filtration and washed with distilled water until the filtrate is free of alkali and 1,3,dichloro-2-propanol. The pulp is then air-dried at room temperature, fiberized in a Pallman fiberizer and wet-laid using a Formet Dynamique wet-forming system. The wet-laid web is then air-dried and found to have a density of 0.09 grams per cubic centimeter. A control sample of non-crosslinked northern spruce kraft pulp is similarly prepared and wet-laid to form a web. The web of non-crosslinked northern spruce kraft pulp is found to have a density of 0.26 grams per cubic centimeter.

The webs so produced are subjected to vertical wicking tests as described above. The results of these tests are set forth in Table 5.

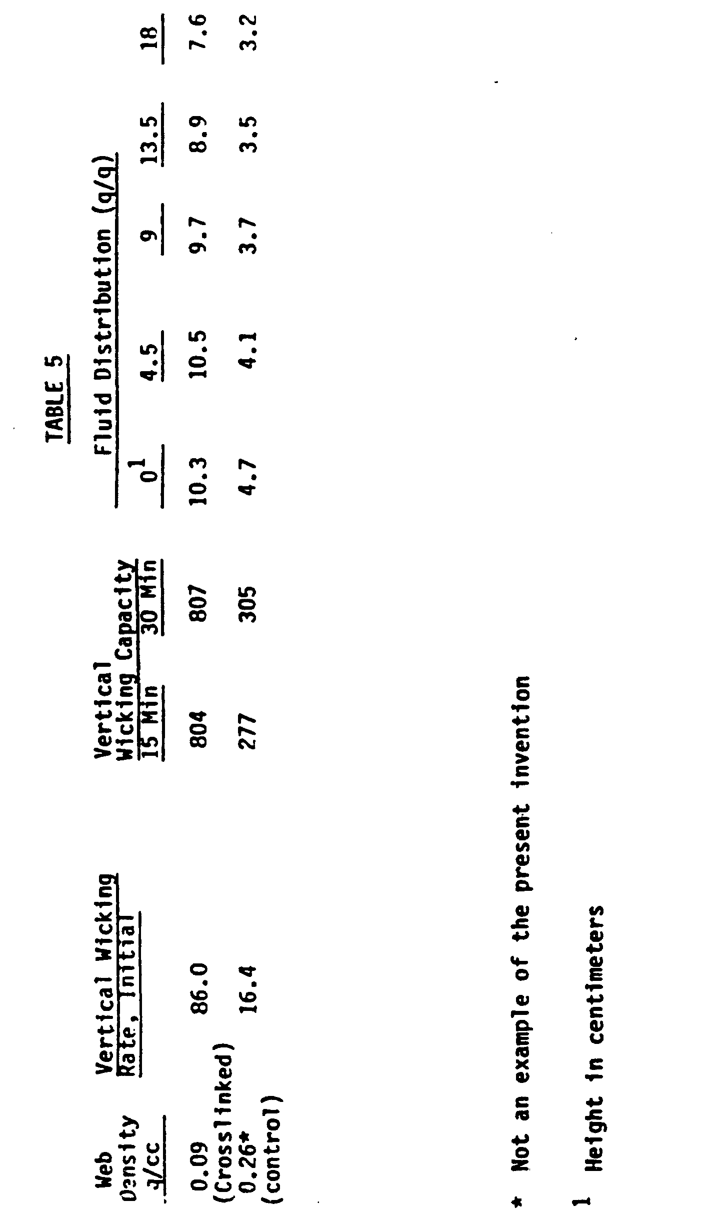

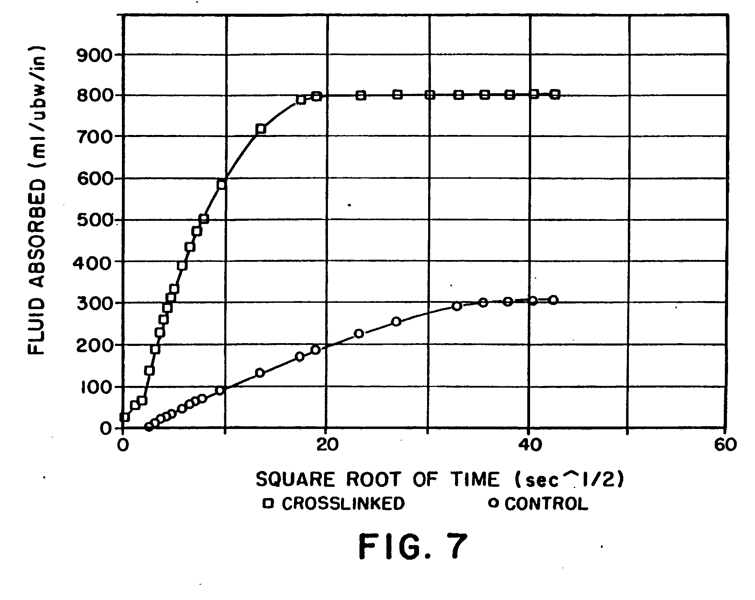

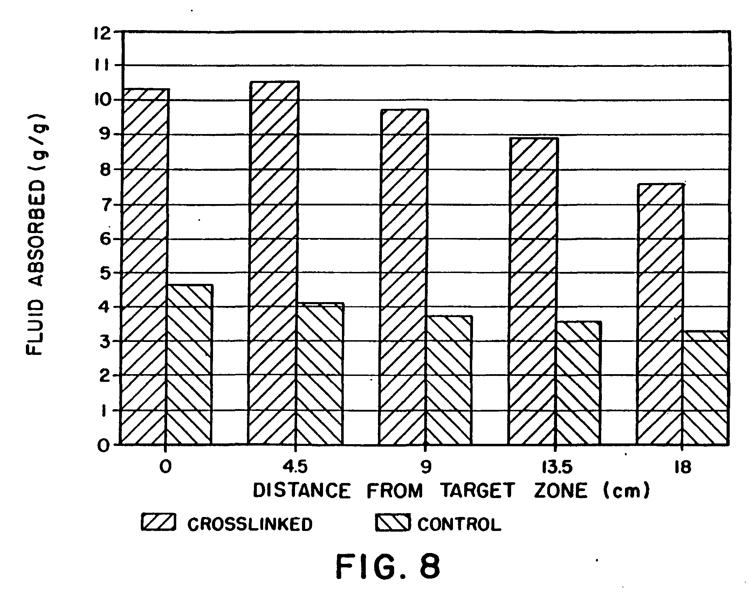

The data set forth in Table 5 concerning the vertical wicking rates is graphically illustrated in Figure 7. The data set forth in Table 5 concerning fluid distribution is graphically illustrated in Figure 8.

As can be seen in reference to Table 5, and Figures 7 and 8, the wet cosslinked, water-laid web has a significantly greater vertical wicking rate than the non-crosslinked water and web. Additionally, the crosslinked water-laid sample is seen to have greater fluid distribution at all measured distances compared to the non-crosslinked water-laid web. Further, it is noted that comparison of the water-laid wet crosslinked web to an air-laid wet crosslinked web of similar density (Table 3, Figure 3, 0.10 density) reveals that the water-laid web has a higher vertical wicking rate, capacity and fluid distribution (18 centimeters).

While it is often necessary to compact air-laid, wet crosslinked fiber webs to within the desired density, ofttimes water-laid, wet crosslinked webs will fall within the desired density range without the necessity of compaction. Nonetheless, it is possible to compact the water-laid webs to increase their density, if this is desirable.

### Example 4

### Sample 1

Into a mixing vessel is placed 200 gram of a low-formaldehyde modified glyoxal resin commercially available from American Hoechst under the trade designation Cassurit TP 2236 and 250 milliliters of an aqueous hydrochloric acid solution (37% HCl). The resulting solution is diluted to a total of 500 milliliters with distilled water. To the 500 milliliters of solution is added 100 grams of southern pine kraft pulp (Example 1) fluff to form a slurry. The slurry is mixed and allowed to stand at room temperature for 30 minutes. The wood pulp fluff is then recovered by filtration and washed with an excess of distilled water. The wood pulp fluff is then neutralized with a sodium carbonate solution (5% sodium carbonate) and washed three more times with distilled water. The pulp is then air-dried. The air-dried pulp is pulverized in a Pallman pulverizer and air-laid on a hand-sheet former to form a fibrous web. A control sample (Control [1]) of non-crosslinked pulp fluff is air-laid on a hand-sheet former to form a fibrous web. The air-laid webs are then densified to a density of 0.15 grams per cubic centimeter in a Dake press.

### Sample 2

Wet crosslinked wood pulp fluff is prepared as described above in connection with sample 1, except that after washing, neutralization with sodium carbonate, and three additional washings, the wood pulp fluff is not air-dried. Instead, the wood pulp fluff is reslurried in distilled water and wet-laid into webs using a Flormet Dynamique wet-forming system. The resulting wet-laid sheets are then air-dried. The wet-laid sheet of wet crosslinked wood pulp fluff is then compressed to a density of 0.15 grams per cubic centimeter in a Dake press. Similarly, a control sample (Control [2]) is prepared by wet-laying non-crosslinked CR 54 wood pulp fluff into a fibrous web. The web is similarly densified to a density of 0.15 grams per cubic centimeter.

The above samples and controls are then subjected to vertical wicking property determinations. The results of the vertical wicking determinations are set forth in table 6.

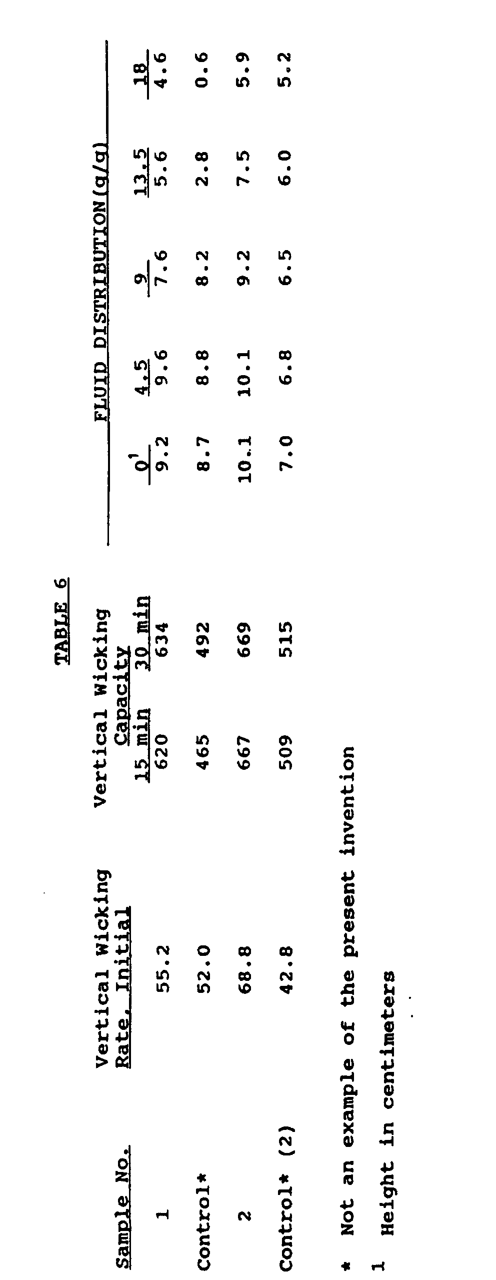

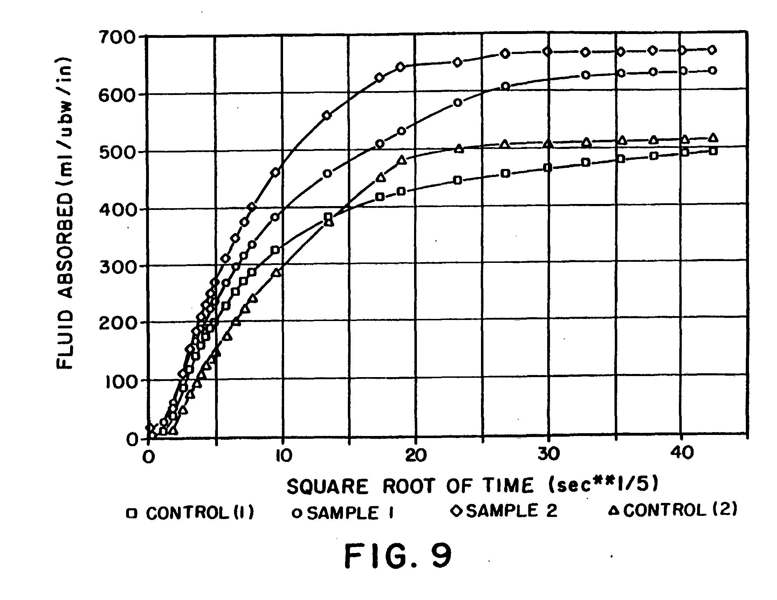

Figure 9 graphically represents the vertical wicking rate of the samples set forth in Example 4. As can be seen from reference to figure 9, the vertical wicking rate for both the air-laid and wet-laid crosslinked pulp is significantly improved compared to the air-laid and wet-laid control samples.

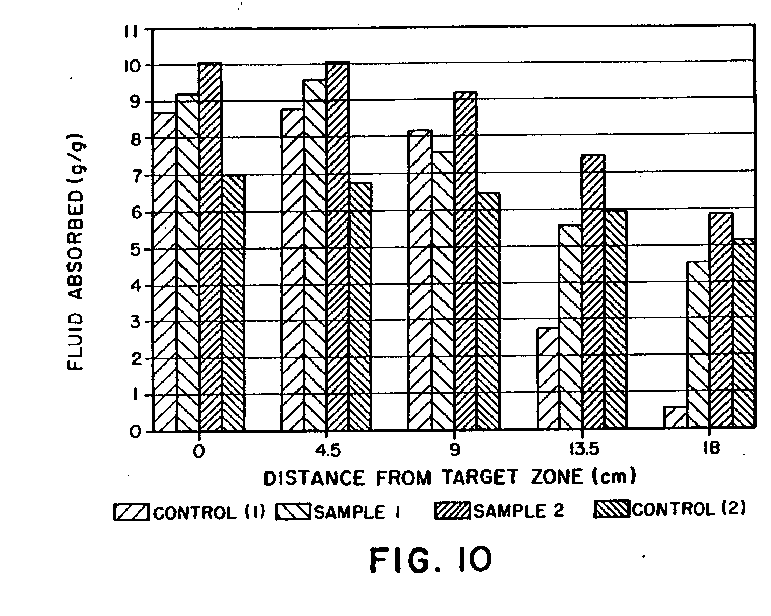

Figure 10 is bar graph illustrating the fluid distribution data set forth in Table 6. As can be seen from reference to Figure 10, the vertical fluid distribution at 18 centimeters for both the air-laid and wet-laid crosslinked pulps is improved compared to the respective control samples.

### Example 5

Bleached southern pine kraft pulp (75% pine softwood, 25% hardwood) is wet crosslinked with N,N'-methylenebisacrylamide (MBA) in the following manner. Two hundred grams of pulp is dispersed in distilled water and filtered. The wet pulp is found, by weighting, to have retained 354 grams (ml) of water. the wet pulp is placed in a plastic bag with 1076 ml of tap water. The plastic bag is sealed and submerged in a 60°C waterbath. After temperature equilibration, an aqueous solution comprising 60 g sodium hydroxide dissolved in 250 ml of water is added to the plastic bag. The pulp is kneaded to distribute the sodium hydroxide solution. To the bag is then added, in portions, 60 grams of N,N'-methylenebisacrylamide. The bag and its contents are kneaded to ensure complete disolution and distribution of the N,N'-methylenebisacrylamide. The bag is placed in the water bath and left undisturbed for 90 minutes.

The pulp is recovered by filtration. The pulp is washed on the filter with 3 liters of tap water. The pulp is then washed an additional eight times by slurrying the pulp in 3 liters of 40-45 °C tap water and filtering. The pulp is then fiberized and allowed to dry on racks under ambient conditions.
After drying, the pulp is fluffed and air-laid on a hand-sheet former to form fibrous webs. Additionally, a control sample of non-crosslinked southern pine kraft pulp is air-laid on a hand-sheet former to form a fibrous web. The air-laid webs are then densified to a density of 0.15 grams per cubic centimeter in a Dake press.

The webs are then subjected to vertical wicking property determinations. The results of the vertical wicking determinations are set forth in Table 7.

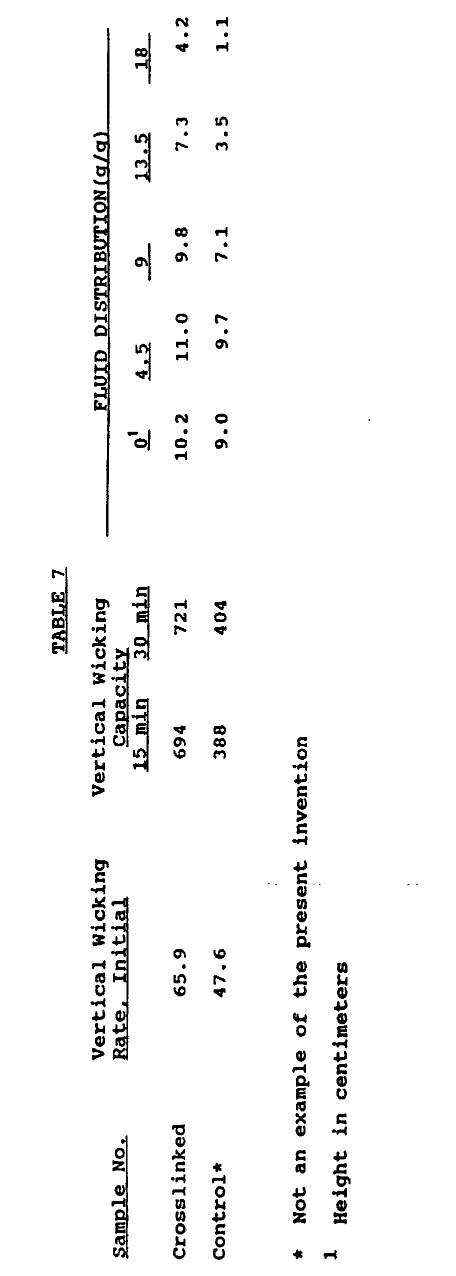

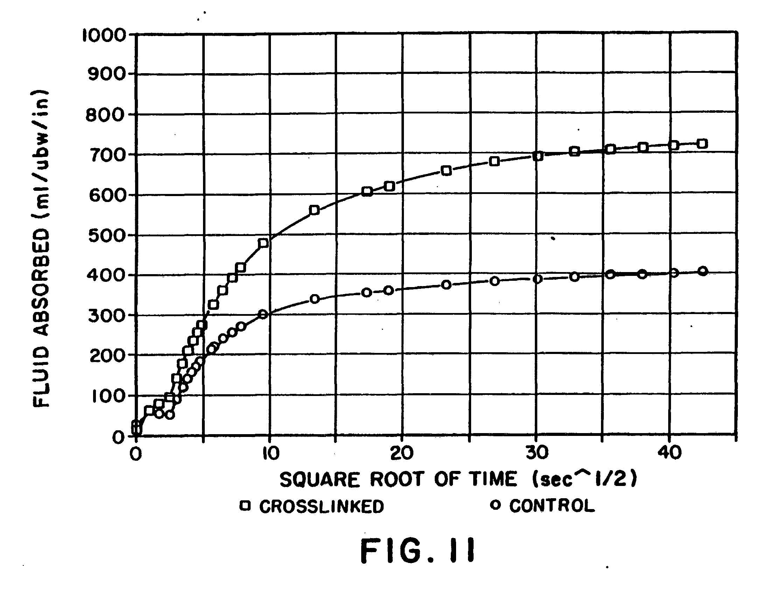

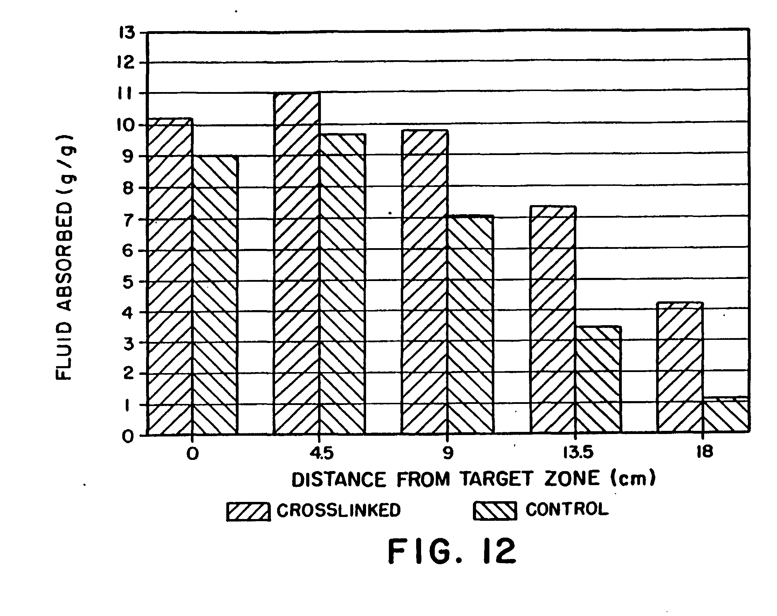

As can be seen from reference to Figures 11 and 12 the vertical wicking properties of the pulp wet crosslinked with the N,N'-methylenebisacrylamide are significantly improved compared to the control sample.

## Claims

1. An absorbent web of wet-crosslinked cellulose fibers, said web having a density within the range of from 0.08 gram per cubic centimeter to 0.35 gram per cubic centimeter, said web being **characterized in that** the crosslinked cellulose fibers are obtainable by crosslinking while the fibers are in a swollen state and **in that** said web possesses improved vertical wicking properties compared to a similar web of nonwet-crosslinked cellulose fibers, wherein at least one of the initial vertical wicking rate, the vertical wicking capacity at 15 or 30 minutes, or the vertical fluid distribuion at 18 centimeters is at least 20 percent, preferably 40 percent, greater than said similar web of nonwet-crosslinked cellulose fibers.

2. The web according to claim 1 wherein the web is air-laid and has a density of from 0.15 to 0.30 gram per cubic centimeter.

3. The web according to claim 1 wherein the web is water-laid and has a density of from 0.08 to 0.3 gram per cubic centimeter.

4. The web of one of claims 1 to 3 wherein at least two of the initial vertical wicking rate, the vertical wicking capacity at 15 or 30 minutes, or the vertical fluid distribution at 18 centimeters is at least 20 percent, preferably 40 percent, greater than said similar web of nonwet-crosslinked cellulose fibers.

5. A web of wet-crosslinked cellulose fibers, said web being **characterized in that** it is obtained by subjecting cellulose fibers to a crosslinking process while said fibers are in a swollen state, drying said fibers, air-laying said wet-crosslinked cellulose fibers into a web and compressing said air-laid web to a density within the range of from 0.10 to 0.35 gram per cubic centimeter, said web possessing improved vertical wicking properties compared to a similar web of nonwet-crosslinked cellulose fibers, wherein at least one of the initial vertical wicking rate, the vertical wicking capacity at 15 or 30 minutes, or the vertical fluid distribution at 18 centimeters is at least 20 percent, preferably 40 percent, greater than said similar web of nonwet-crosslinked cellulose fibers.

6. The web according to claim 5 wherein the web is compressed to a density within the range of from 0.15 to 0.3 gram per cubic centimeter.

7. The web according to claim 5 or 6 wherein the web demonstrates improved wet resiliency such that the specific volume (in cubic centimeters per gram) of the web when wet is at least 45 percent greater than the specific volume of the web when dry, said specific volumes being determined under a 0.0138 bar load.

8. The web of one of the preceding claims further comprising an amount of a water-swellable polymeric material in flow communication therewith.

9. Use of the web according to one of the preceding claims as an absorbent product, especially as a diaper.

10. An absorbent product, said product comprising an absorbent web of wet-crosslinked cellulose fibers, said web having a density within the range of from 0.08 to 0.35 gram per cubic centimeter, said web being **characterized in that** the crosslinked cellulose fibers are obtainable by crosslinking while the fibers are in a swollen state and **in that** said web possesses improved vertical wicking properties compared to a similar web of nonwet-crosslinked cellulose fibers, wherein at least one of the initial vertical wicking rate, the vertical wicking capacity at 15 or 30 minutes, or the vertical fluid distribution at 18 centimeters is at least 20 percent, preferably 40 percent, greater than said similar web on nonwet-crosslinked cellulose fibers.

11. The absorbent product of claim 10 wherein the absorbent product further comprises an amount of a water-swellable polymeric material in flow communication with said absorbent web.

12. The absorbent product according to claim 11 wherein the absorbent product is a diaper.

## Patentansprüche

1. Saugfähige Bahn aus nassvernetzten Zellulosefasern, wobei die Bahn eine Dichte im Bereich von 0,08 Gramm pro Kubikzentimeter bis 0,35 Gramm pro Kubikzentimeter aufweist, wobei die Bahn **dadurch gekennzeichnet ist, dass** die vernetzten Zellulosefasern durch Vernetzung erhältlich sind, während sich die Fasern in einem gequollenen Zustand befinden, und dass die Bahn im Vergleich zu einer entsprechenden Bahn aus nicht nassvernetzten Zellulosefasern verbesserte vertikale Saugeigenschaften besitzt, wobei mindestens eine Eigenschaft, von anfänglicher vertikaler Sauggeschwindigkeit, vertikaler Saugfähigkeit nach 15 oder 30 Minuten oder vertikaler Flüssigkeitsverteilung bei 18 Zentimetern mindestens 20 Prozent, vorzugsweise 40 Prozent größer ist als bei der entsprechenden Bahn aus nicht nassvernetzten Zellulosefasern.

2. Bahn nach Anspruch 1, wobei die Bahn luftabgelegt ist und eine Dichte von 0,15 bis 0,30 Gramm pro Kubikzentimeter aufweist.

3. Bahn nach Anspruch 1, wobei die Bahn wasserabgelegt ist und eine Dichte von 0,08 bis 0,3 Gramm pro Kubikzentimeter aufweist.

4. Bahn nach einem der Ansprüche 1 bis 3, wobei mindestens zwei Eigenschaften, von anfänglicher vertikaler Sauggeschwindigkeit, vertikaler Saugfähigkeit nach 15 oder 30 Minuten oder vertikaler Flüssigkeitsverteilung bei 18 Zentimetern mindestens 20 Prozent, vorzugsweise 40 Prozent größer sind als bei der entsprechenden Bahn aus nicht nassvernetzten Zellulosefasern.

5. Bahn aus nassvernetzten Zellulosefasern, wobei die Bahn **dadurch gekennzeichnet ist, dass** sie erhalten wird indem Zellulosefasern einem Vernetzungsprozess unterworfen werden während sich die Fasern in einem gequollenen Zustand befinden, die Fasern getrocknet werden, die nassvernetzten Zellulosefasern zu einer Bahn luftabgelegt werden und die luftabgelegte Bahn zu einer Dichte im Bereich von 0,10 bis 0,35 Gramm pro Kubikzentimeter komprimiert wird, wobei die Bahn im Vergleich zu einer entsprechenden Bahn aus nicht nassvernetzten Zellulosefasern verbesserte vertikale Saugeigenschaften besitzt, wobei mindestens eine Eigenschaft, von anfänglicher vertikaler Sauggeschwindigkeit, vertikaler Saugfähigkeit nach 15 oder 30 Minuten oder vertikaler Flüssigkeitsverteilung bei 18 Zentimetern mindestens 20 Prozent, vorzugsweise 40 Prozent größer ist als bei der entsprechenden Bahn aus nicht nassvernetzten Zellulosefasern.

6. Bahn nach Anspruch 5, wobei die Bahn auf eine Dichte im Bereich von 0,15 bis 0,3 Gramm pro Kubikzentimeter komprimiert ist.

7. Bahn nach Anspruch 5 oder 6, wobei die Bahn eine verbesserte Nasselastizität aufweist, so dass das spezifische Volumen (in Kubikzentimetern pro Gramm) der Bahn im nassen Zustand mindestens um 45 Prozent größer als das spezifische Volumen der Bahn im trockenen Zustand ist, wobei diese spezifischen Volumina unter einer Belastung von 0,0138 bar bestimmt werden.

8. Bahn nach einem der vorhergehenden Ansprüche, welche ferner eine mit dieser in Fliessverbindung stehenden Menge eines wasserquellbaren polymeren Materials enthält.

9. Verwendung der Bahn nach einem der vorhergehenden Ansprüche als saugfähiges Produkt, insbesondere als Windel.

10. Saugfähiges Produkt, wobei das Produkt eine Bahn aus nassvernetzten Zellulosefasern umfasst, wobei die Bahn eine Dichte im Bereich von 0,08 Gramm bis 0,35 Gramm pro Kubikzentimeter aufweist, wobei die Bahn **dadurch gekennzeichnet ist, dass** die vernetzten Zellulosefasern durch Vernetzung erhältlich sind, während sich die Fasern in einem gequollenen Zustand befinden, und dass die Bahn im Vergleich zu einer entsprechenden Bahn aus nicht nassvernetzten Zellulosefasern verbesserte vertikale Saugeigenschaften besitzt, wobei mindestens eine Eigenschaft, von anfänglicher vertikaler Sauggeschwindigkeit, vertikaler Saugfähigkeit nach 15 oder 30 Minuten oder vertikaler Flüssigkeitsverteilung bei 18 Zentimetern mindestens 20 Prozent, vorzugsweise 40 Prozent größer ist als bei der entsprechenden Bahn aus nicht nassvernetzbaren Zellulosefasern.

11. Saugfähiges Produkt nach Anspruch 10, wobei das saugfähige Produkt ferner eine Menge eines wasserquellbaren polymeren Materials enthält, das mit der saugfähigen Bahn in Fliessverbindung steht.

12. Saugfähiges Produkt nach Anspruch 11, wobei das saugfähige Produkt eine Windel ist.

## Revendications

1. Nappe absorbante de fibres de cellulose réticulée à l'état humide, ladite nappe ayant une masse spécifique comprise dans la gamme allant de 0,08 g par centimètre cube à 0,35 g par centimètre cube, ladite nappe étant **caractérisée en ce que** les fibres de cellulose réticulée peuvent être obtenues par réticulation tandis que les fibres sont à l'état gonflé et **en ce que** ladite nappe possède des propriétés de drainage vertical améliorées par comparaison à celles d'une nappe similaire de fibres de cellulose non réticulée à l'état humide, et dans laquelle l'une au moins des propriétés suivantes : la vitesse de drainage vertical initiale, la capacité de drainage vertical en 15 ou 30 minutes ou la distribution de fluide verticale à 18 cm, est au moins de 20%, de préférence de 40%, supérieure à celle de ladite nappe similaire de fibres de cellulose non réticulée à l'état humide.

2. Nappe selon la revendication 1, dans laquelle la nappe est étalée à l'air et a une masse spécifique comprise entre 0,15 et 0,30 g par centimètre cube.

3. Nappe selon la revendication 1, dans laquelle la nappe est étalée à l'eau et a une masse spécifique comprise entre 0,08 et 0,3 g par centimètre cube.

4. Nappe selon l'une des revendications 1 à 3, dans laquelle au moins deux des propriétés suivantes : la vitesse de drainage vertical initiale, la capacité de drainage vertical en 15 ou 30 minutes, ou la distribution de fluide verticale à 18 centimètres, sont au moins de 20%, et de préférence de 40%, supérieures à celles de ladite nappe similaire de fibres de cellulose non réticulée à l'état humide.

5. Nappe de fibres de cellulose réticulée à l'état humide, ladite nappe étant **caractérisée en ce qu'**elle est obtenue en soumettant des fibres de cellulose à un procédé de réticulation tandis que lesdites fibres sont à l'état gonflé, à sécher lesdites fibres, à étaler à l'air lesdites fibres de cellulose réticulée à l'état humide en une nappe et à comprimer ladite nappe étalée à l'air à une masse spécifique comprise dans la gamme allant de 0,10 à 0,35 g par centimètre cube, ladite nappe présentant des propriétés de drainage vertical améliorées par comparaison à celles d'une nappe similaire de fibres de cellulose non réticulée à l'état humide, l'une au moins des propriétés suivantes : la vitesse de drainage vertical initiale, la capacité de drainage vertical en 15 ou 30 minutes ou la distribution de fluide verticale à 18 centimètres, est au moins de 20%, et de préférence de 40%, supérieure à celle de ladite nappe similaire de fibres de cellulose non réticulée à l'état humide.

6. Nappe selon la revendication 5, dans laquelle la nappe est comprimée à une masse spécifique comprise dans la gamme allant de 0,15 à 0,3 g par centimètre cube.

7. Nappe selon la revendication 5 ou 6, dans laquelle la nappe montre une résilience améliorée à l'état humide telle que le volume spécifique (en centimètres cubes par gramme) de la nappe, lorsqu'elle est humide, est au moins de 45% supérieur au volume spécifique de la nappe lorsqu'elle est sèche, lesdits volumes spécifiques étant déterminés sous une charge de 0,0138 bar.

8. Nappe selon l'une des revendications précédentes, comprenant en outre une quantité de matériau polymère gonflable à l'eau en communication d'écoulement avec celle-ci.

9. Utilisation de la nappe selon l'une des revendications précédentes dans un produit absorbant, en particulier un change pour bébé.

10. Produit absorbant, ledit produit comprenant une nappe absorbante de fibres de cellulose réticulée à l'état humide, ladite nappe ayant une masse spécifique comprise dans la gamme allant de 0,08 à 0,35 g par centimètre cube, ladite nappe étant **caractérisée en ce que** les fibres de cellulose réticulée peuvent être obtenues par réticulation tandis que les fibres sont à l'état gonflé et **en ce que** ladite nappe possède des propriétés de drainage vertical améliorées par comparaison à celles d'une nappe similaire de fibres de cellulose non réticulée à l'état humide, l'une au moins des propriétés suivantes : la vitesse de drainage vertical initiale, la capacité de drainage vertical en 15 ou 30 minutes, ou la distribution de fluide verticale à 18 centimètres est au moins de 20%, et de préférence de 40%, supérieure à celle de ladite nappe similaire de fibres de cellulose non réticulée à l'état humide.

11. Produit absorbant selon la revendication 10, dans lequel le produit absorbant comprend en outre une quantité de matériau polymère gonflable à l'eau en communication d'écoulement avec ladite nappe absorbante.

12. Produit absorbant selon la revendication 11, qui est un change pour bébé.
